(19) Europäisches Patentamt

European Patent Office

Office européen des brevets

(11) **EP 2 042 516 A1**

(12) **EUROPEAN PATENT APPLICATION**

(43) Date of publication:
**01.04.2009 Bulletin 2009/14**

(51) Int Cl.:
***C07K 14/52*** *(2006.01)*

(21) Application number: **07450166.9**

(22) Date of filing: **27.09.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **Protaffin Biotechnologie AG
8020 Graz (AT)**

(72) Inventors:
• **Kungl, Andreas
8045 Graz (AT)**
• **Piccinini, Anna Maria
8010 Graz (AT)**
• **Weber, Christian
52074 Aachen (DE)**

(74) Representative: **Sonn & Partner Patentanwälte
Riemergasse 14
1010 Wien (AT)**

(54) **Glycosaminoglycan-antagonising MCP-1 mutants and methods of using same**

(57) Novel mutants of human monocyte chemoattractant protein 1 (MCP-1) with increased glycosaminoglycan (GAG) binding affinity and knocked-out or reduced GPCR activity compared to wild type MCP-1, and their use for therapeutic treatment of inflammatory diseases.

**Description**

The present invention relates to novel mutants of human monocyte chemoattractant protein 1 (MCP-1) with increased glycosaminoglycan (GAG) binding affinity and knocked-out or reduced GPCR activity compared to wild type MCP-1, and to their use for therapeutic treatment of inflammatory diseases.

All chemokines, with the exception of lymphotactin and fraktaline/neurotactin which are members of the C and CX3C chemokine subfamily, respectively, have four cysteines in conserved positions and can be divided into the CXC or α-chemokine and the CC or β-chemokine subfamilies on the basis of the presence or absence, respectively, of an amino acid between the two cysteines within the N-terminus. Chemokines are small secreted proteins that function as intercellular messengers to orchestrate activation and migration of specific types of leukocytes from the lumen of blood vessels into tissues (Baggiolini M., J. Int. Med. 250, 91-104 (2001)). This event is mediated by the interaction of chemokines with seven transmembrane G-protein-coupled receptors (GPCRs) on the surface of target cells. Such interaction occurs in vivo under flow conditions. Therefore, the establishment of a local concentration gradient is required and ensured by the interaction of chemokines with cell surface glycosaminoglycans (GAGs). Chemokines have two major sites of interaction with their receptors, one in the N-terminal domain which functions as a triggering domain, and the other within the exposed loop after the second cysteine, which functions as a docking domain (Gupta S.K. et al., Proc.Natl.Acad.Sci., USA, 92, (17), 7799-7803 (1995)). The GAG binding sites of chemokines comprise clusters of basic amino acids spatially distinct (Ali S. et al., Biochem.J. 358, 737-745 (2001)). Some chemokines, such as RANTES, have the BBXB motif in the 40s loop as major GAG binding site; IL-8 interacts with GAGs through the C-terminal α-helix and Lys 20 in the proximal N-loop. Other chemokines, such as MCP-1, show a significant overlap between the residues that comprise the receptor binding and the GAG binding site (Lau E.K. et al., J. Biol. Chem., 279 (21), 22294-22305 (2004)).

In the context of the chemokine-β family of cytokines, monocyte chemoattractant protein-1 (MCP-1) is a monocyte and lymphocyte-specific chemoattractant and activator found in a variety of diseases that feature a monocyte-rich inflammatory component, such as atherosclerosis (Nelken N.A. et al., J. Clin. Invest. 88, 1121-1127 (1991); Yla-Herttuala, S., Proc. Natl. Acad. Sci USA 88, 5252-5256 (1991), rheumatoid arthritis (Koch A.E. et al., J. Clin. Invest. 90, 772-779 (1992); Hosaka S. et al., Clin. Exp. Immunol. 97(3), 451-457 (1994), Robinson E. et al., Clin. Exp. Immunol. 101(3), 398-407 (1995)), inflammatory bowel disease (MacDermott R.P. et al., J. Clin. Immunol. 19, 266-272 (1999)) and congestive heart failure (Aukrust P., et al., Circulation 97, 1136-1143 (1998), Hohensinner P.J. et al., FEBS Letters 580, 3532-3538 (2006)). Crucially, knockout mice that lack MCP-1 or its receptor CCR2, are unable to recruit monocytes and T-cells to inflammatory lesions (Grewal I.S. et al., J. Immunol. 159 (1), 401-408 (1997), Boring L. et al., J. Biol. Chem. 271 (13), 7551-7558 (1996), Kuziel W.A., et al., Proc.Natl.Acad.Sci. USA 94 (22), 12053-8 (1997), Lu B., et al., J. Exp.Med. 187 (4), 601-8 (1998)); furthermore, treatment with MCP-1 neutralizing antibodies or other biological antagonists can reduce inflammation in several animal models (Lukacs N.W. et al., J.Immunol., 158 (9), 4398-4404 (1997), Flory C.M. et al., 1. Lab. Invest. 69 (4), 396-404 (1993), Gong J.H., et al., J.Exp.Med. 186 (1), 131-7 (1997), Zisman D.A. et al., J.Clin.Invest. 99 (12), 2832-6 (1997)). Finally, LDL-receptor/MCP-1-deficient and apoB-transgenic/MCP-1-deficient mice show considerably less lipid deposition and macrophage accumulation throughout their aortas compared to the WT MCP-1 strains (Alcami A. et al., J. Immunol. 160 (2), 624-33 (1998), Gosling J. et al., J. Clin. Invest. 103 (6), 773-8 (1999)).

Since the first chemokines and their receptors have been identified, the interest on exactly understanding their roles in normal and diseased physiology has become more and more intense. The constant need for new anti-inflammatory drugs with modes of action different from those of existing drugs support the development of new protein-based GAG-antagonists and their use in an inflammatory set.

Since in the last years the molecular basis of the interactions of MCP-1 with CCR2 and GAGs have been studied in great detail, targeted engineering of the chemokine towards becoming an effective antagonist of MCP-1's biological action is feasible. For this purpose, the inventors could generate several recombinant MCP-1 variants that compete with their wild type counterpart for glycosaminoglycan binding and show reduced or knocked out activation of leukocytes.

Consquently, one subject matter of the present invention is to inhibit leukocyte, more specifically monocyte and T cell, migration by antagonizing the GAG interaction with an MCP-1-based mutant protein in the context of inflammatory or allergic processes.

The invention is based on engineering a higher GAG binding affinity into human MCP-1, either by modifying the wild type GAG binding region or by introducing a new GAG binding region into the MCP1 protein and simultaneously knocking out or reducing its GPCR activity, specifically the CCR2 activity of the chemokine. This has been successfully accomplished with a mutant MCP-1 protein wherein a region of the MCP-1 protein is modified in a structure conserving way by introducing basic and/or electron donating amino acids or replacing native amino acids with basic and/or electron donating amino acids and modifying the N-terminal region of said MCP-1 protein by addition, deletion and/or replacement of amino acids and, optionally, adding an N-terminal Methionine to the mutant MCP-1 protein, resulting in partial or complete loss of chemotactic activity. Said inventive MCP-1 mutants can preferably exhibit a minimum five-fold improved Kd for standard GAGs (heparin or heparan sulfate) and they are deficient or reduced in inducing Calcium-release in standard monocytic cell culture.

Further, the present invention provides an isolated polynucleic acid molecule coding for the mutant MCP-1 protein of the invention, and a vector comprising an isolated DNA molecule coding for the mutant MCP-1 protein, and a recombinant cell transfected with the vector.

The mutant MCP-1 protein according to the present invention can also be formulated as a pharmaceutical composition comprising the mutant MCP-1 protein or a polynucleic acid molecule coding for MCP-1 mutant protein, a vector containing an isolated DNA molecule coding for the MCP-1 mutant protein, and a pharmaceutically acceptable carrier.

Said MCP-1 mutant protein or the polynucleotide coding therefor or the vector containing said polynucleotide can also be used for inhibiting or suppressing the biological activity of the respective wild type protein.

The inventive MCP-1 mutant protein according to the invention, a polynucleic acid coding therefor or a vector containing the polynucleotide can also be used in a method for preparing a medicament for the treatment of chronic or acute inflammatory diseases or allergic conditions. Preferably, the disease is selected for the group comprising rheumatoid arthritis, uveitis, inflammatory bowel disease, myocardial infarction, congested heart failure or ischemia reperfusion injury.

**Figures:**

Figure 1: Sequence of MCP-1 mutants, mutations with respect to the wild type chemokine are underlined

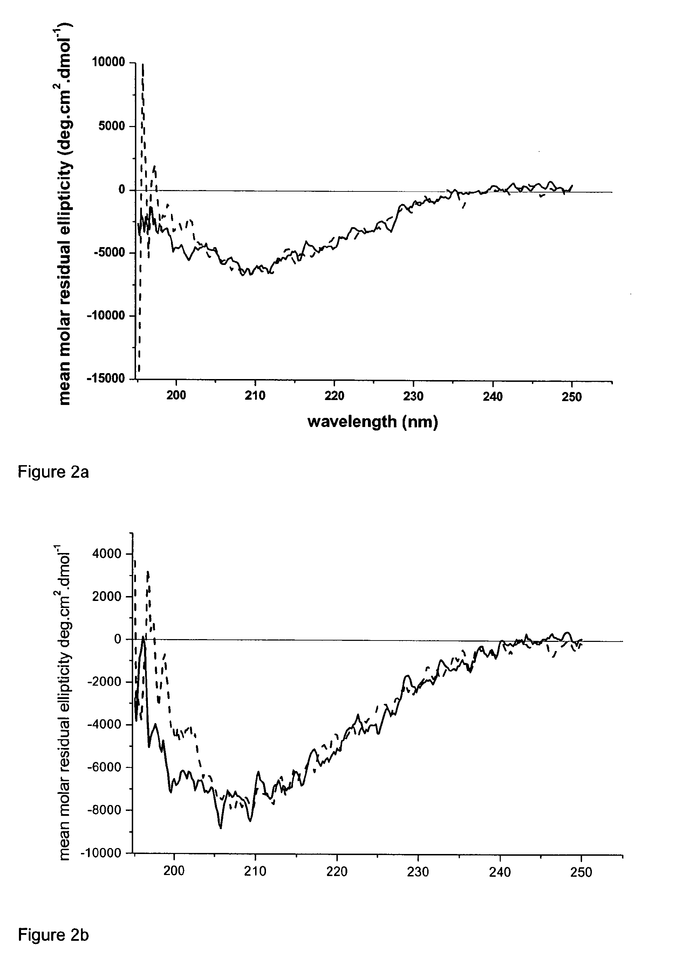

Figure 2: Structural change of wtMCP-1 (Figure 2 a) and Met-MCP-1 Y13A S21 K Q23R (Figure 2b) upon heparan sulfate binding, as shown by far-UV CD spectroscopy

Figure 3: Scatchard plot analysis and equilibrium dissociation constants (Kd values) of WT MCP-1 (solid squares), Met-MCP-1 Y13A S21 K (solid triangles) and Met-MCP-1 Y13A S21 K Q23R (open circles) binding to unfractionated HS

Figure 4: Calcium influx assay induced by 20 nM wtMCP-1 and MCP-1 mutants (20nM each) on THP-1 cells. The changes in fluorescence emission at 495nm due to calcium mobilization induced by addition of chemokines are displayed: wtMCP-1 (A), Met-MCP-1 Y13A S21 K (B), Met-MCP-1 Y13A S21 K Q23R (C) and Met-MCP-1 Y13A S21 K Q23R V47K (D).

Figure 5: Chemotaxis of THP-1 cells induced by wtMCP-1 and MCP-1 mutants at a concentration of 10nM (error bars represent the SEM of three independent experiments). 1 wtMCP-1, 2 Met-MCP-1, 3 Met-MCP-1 Y13A S21 K, 4 Met-MCP-1 Y13A S21 K Q23R, 5 Met-MCP-1 Y13A S21 K Q23R V47K.

Figure 6: Dose-dependent inhibition of monocyte adhesion/efflux by Met-MCP-1 Y13AS21KQ23R (described by the compound code PA05-008) as measured in a murine *ex vivo* carotide injury model.

Figure 7: Improvement of clinical and histological scores in a rat model of auto-immune uveitis after treatment with Met-MCP-1 Y13AS21KQ23R.

Figure 8: Effect of Met-MCP-1 Y13AS21KQ23R (indicated as PA008) on ischemia reperfusion injury in a murine myocardial infarct model.

All dimensions specified in this disclosure are by way of example only and are not intended to be limiting. Further, the proportions shown in the foregoing figures are not necessarily to scale.

It has been shown that increased GAG binding affinity can be introduced by increasing the relative amount of basic and/or electron donating amino acids in the GAG binding region (also described in WO 05/054285, incorporated in total herein by reference), leading to a modified protein that acts as competitor with natural GAG binding proteins. This was particularly shown for interleukin-8. The specific location of GAG binding regions and their modification by selectively introducing basic and/or electron donating amino acids was not disclosed for MCP-1 protein.

Additionally, the amino terminus of MCP-1 was found to be essential for chemokine signalling through its GPC receptor CCR2.

The invention now provides a novel MCP1 mutant protein with increased GAG binding affinity and reduced GPCR activity compared to the wild type MCP1 protein, wherein a region of the MCP-1 protein is modified in a structure conserving way by insertion of at least one basic and/or electron donating amino acid or by replacement of at least one non-basic amino acid by at least one basic and/or electron donating amino acid and at least one amino acid of the first 1 to 10 amino acids of the N-terminal region of said MCP-1 protein is further modified by addition, deletion and/or replacement of at least one amino acid residue. According to the definition as used in the present application MCP-1 mutant protein also includes any parts or fragments thereof that still show chemokine activity like monocyte or T-cell chemotaxis and Carelease.

A protocol for introducing or improving a GAG binding site is, for example, partially described in WO 05/054285 and can be as follows:

- Identify a region of the protein which is involved in GAG binding
- Design a new GAG binding site by introducing (replacement or insertion) basic or electron donating amino acids, preferably Arg, Lys, His, Asp and Gln residues at any position or by deleting amino acids which interfere with GAG binding
- Check the conformational stability of the resulting mutant protein in silica
- Clone the wild type protein cDNA (alternatively: purchase the cDNA)
- Use this as template for PCR-assisted mutagenesis to introduce the above mentioned changes into the amino acid sequence
- Subclone the mutant gene into a suitable expression system (prokaryotic or eukaryotic dependent upon biologically required post-translational modifications)
- Expression, purification and characterization of the mutant protein in vitro Criterion for an increased GAG binding affinity: $K_d^{GAG}$(mutant) ≤ 10uM.
- Check for structural conservation by far-UV CD spectroscopy or 1-D NMR spectroscopy.
  A deviation of the modified structure as measured by far-UV CD spectroscopy from wild type structure of less than 30%, preferably less than 20%, is defined as structure conserving modification according to the invention.

According to the present invention the MCP-1 mutant protein may comprise a modification in a structure conserving way wherein the deviation of the modified structure as measured by far-UV CD spectroscopy from wild type MCP1 structure is less than 30%, preferably less than 20%.
According to an alternative embodiment, the structure conserving modification is located not within the N-terminus of the MCP1 protein.

The key residues relating to the GAG binding site of wtMCP-1 are Arg18, Arg24, Lys19, Lys49 with additional contributions from Lys58 and His66. According to the invention, the MCP-1 mutant protein may contain at least one modified amino acid at position 18, 24, 19, 49, 58 and/or 66. Alternatively, the inventive MCP-1 mutant protein may contain an amino acid modification within at least one amino acid at position 21, 23 and/or 47.

The modifications can be, for example, a substitution of, or replacement by, at least one basic or electron donating amino acid. Electron donating amino acids are those amino acids which donate electrons or hydrogen atoms (Droenstedt definition). Preferably, these amino acids can be Asn or Gln. Basic amino acids can be selected from the group consisting of Arg, Lys or His.

More specifically, the MCP-1 mutant protein of the invention is characterized in that Tyr at position 13 is substituted by Ala. Tyr 13 (Y13) was shown to be also a critical residue for signalling, and the replacement of this residue by alanine gave rise to a protein unable to induce chemotaxis. Two-dimensional 1 H-15N HSQC spectra recorded on both deletion and substitution MCP-1 variants revealed that these mutations do not generate misfolded proteins (Chad D. Paavola et al., J. Biol. Chem., 273 (50), 33157-33165 (1998)). Furthermore, the N-terminal methionine reduces the binding affinity of MCP-1 for CCR2 on THP-1 cells (Hemmerich S. et al, Biochemistry 38 (40), 13013-13025 (1999)) so that the chemotactic potency of [Met]-MCP-1 is approximately 300-fold lower than of the wild type (Jarnagin K. et al., Biochemistry 38, 16167-16177 (1999)). This is in contrast to the potent receptor antagonist [Met]-RANTES which does not induce chemotaxis but binds with high affinity to the receptor.

Therefore, according to an alternative embodiment of the invention, the MCP-1 mutant protein may contain an N-terminal Met. MCP-1 variants retaining the N-terminal methionine appear to have an increased apparent affinity for heparin (Lau E.K. et al., J. Biol. Chem. 279 (21), 22294-22305 (2004)). According to the present invention, the N-terminal region of the wild type MCP1 region that can be modified comprises the first 1 to 10 N-terminal amino acids. The inventive MCP-1 mutant protein can also have the N-terminal amino acid residues 2-8 deleted. Truncation of residues 2-8 ([1+9-76] hMCP-1) produces a protein that cannot induce chemotaxis. Specifically, MCP-1 mutant protein can be selected from the group of Met-MCP-1 Y13A S21 K, Met-MCP-1 Y13A S21 K V47K, Met-MCP-1 Y13A S21 K Q23R and Met-MCP-1 Y13A S21 K Q23R V47K.

In order to knock out GPCR activity and at the same time to improve affinity for GAGs, minimizing the number of modifications as far as possible, site-directed MCP-1 mutants were designed using bioinformatical and biostructural tools. This means, since the structure of wtMCP-1 is known, mutants were rationally designed. This means for knocking-in higher GAG binding affinity, that more GAG binding sites are introduced into the already existing GAG binding site by replacing amino acids which are not involved in GAG binding, which are structurally less important, and which are solvent exposed by basic amino acids such as Lysine or Arginine. By doing so, special attention was drawn to conserving the specific GAG interaction sites of MCP-1, i.e. those amino acids responsible for hydrogen bonding and van der Waals contacts with the GAG ligand, as well as the overall fold of the chemokine because we want to preserve the ability of the chemokine to penetrate chemokine networks which relies on protein-protein interactions contained in the surface of MCP-1.

The amino acid sequence of the modified MCP-1 molecule can be alternatively described by the general formula: (M)$_n$Q(PDAINAP)$_m$VTCC(X1)NFTN RKI(X2)V(X3)RLAS YRRITSSKCP KEAVIFKTI(X4) AKEICADPKQ KWVQDSMDHL DKQTQTPKT

wherein X1 is selected from the group consisting of Y and/or A, preferably it is A,

wherein X2 is selected from the group consisting of S, R, K, H, N and/or Q, preferably it is K,

wherein X3 is selected from the group consisting of R, K, H, N and/or Q, preferably it is R,

wherein X4 is selected from the group consisting of V, R, K, H, N and/or Q, preferably it is K, and wherein n and/or m can be either 0 or 1.

A further aspect of the present invention is an isolated polynucleic acid molecule which codes for the inventive protein as described above. The polynucleic acid may be DNA or RNA. Thereby the modifications which lead to the inventive MCP-1 mutant protein are carried out on DNA or RNA level. This inventive isolated polynucleic acid molecule is suitable for diagnostic methods as well as gene therapy and the production of inventive MCP-1 mutant protein on a large scale.

Still preferred, the isolated polynucleic acid molecule hybridizes to the above defined inventive polynucleic acid molecule under stringent conditions. Depending on the hybridisation conditions, complementary duplexes form between the two DNA or RNA molecules, either by perfectly matching or also by comprising mismatched bases (see Sambrook et al., Molecular Cloning: A laboratory manual, 2nd ed., Cold Spring Harbor, N.Y. 1989). Probes greater in length than about 50 nucleotides may accomplish up to 25 to 30% mismatched bases. Smaller probes will accomplish fewer mismatches. The tendency of a target and probe to form duplexes containing mismatched base pairs is controlled by the stringency of the hyridization conditions which itself is a function of factors, such as the concentrations of salt or formamide in the hybridization buffer, the temperature of the hybridization and the post-hybridization wash conditions. By applying well known principles that occur in the formation of hybrid duplexes, conditions having the desired stringency can be achieved by one skilled in the art by selecting from among a variety of hybridization buffers, temperatures and wash conditions. Thus, conditions can be selected that permit the detection of either perfectly matching or partially matching hybrid duplexes. The melting temperature (Tm) of a duplex is useful for selecting appropriate hybridisation conditions. Stringent hybridization conditions for polynucleotide molecules over 200 nucleotides in length typically involve hybridizing at a temperature 15-25°C below the melting temperature of the expected duplex. For olignucleotide probes over 30 nucleotides which form less stable duplexes than longer probes, stringent hybridization usually is achieved by hybridizing at 5 to 10°C below the Tm. The Tm of a nucleic acid duplex can be calculated using a formula based on the percent G+C contained in the nucleic acids and that takes chain lengths into account, such as the formula

$$Tm = 81.5 - 16.6(\log[NA^+]) + 0.41\ (\%\ G+C) - (600/N),$$

where N = chain length.

A further aspect relates to a vector comprising an isolated DNA molecule according to the present invention, as defined above. The vector comprises all regulatory elements necessary for efficient transfection as well as efficient expression of proteins. Such vectors are well known in the art and any suitable vector can be selected for this purpose.

A further aspect of the present invention relates to a recombinant cell which is transfected with an inventive vector as described above. Transfection of cells and cultivation of recombinant cells can be performed as well known in the art. Such a recombinant cell as well as any descendant cell therefrom comprises the vector. Thereby, a cell line is provided which expresses the MCP-1 mutant protein either continuously or upon activation depending on the vector.

A further aspect of the invention relates to a pharmaceutical composition comprising a MCP-1 mutant protein, a polynucleic acid or a vector according to the present invention, as defined above, and a pharmaceutically acceptable carrier. Of course, the pharmaceutical composition may further comprise additional substances which are usually present in pharmaceutical compositions, such as salts, buffers, emulgators, coloring agents, etc.

A further aspect of the present invention relates to the use of the MCP-1 protein, a polynucleic acid or a vector according to the present invention, as defined above, in a method for either in vivo or in vitro inhibiting or suppressing the biological activity of the respective wild type protein. As mentioned above, the MCP-1 mutant protein of the invention will act as an antagonist whereby the side effects which occur with known recombinant proteins will not occur with the inventive MCP-1 mutant protein. In this case this will particularly be the biological activity involved in inflammatory reactions.

Therefore, a further use of the MCP-1 protein, a polynucleic acid or a vector according to the present invention, as defined above, is in a method for producing a medicament for the treatment of an inflammatory condition. In particular, it will act as antagonist without side effects and will be particularly suitable for the treatment of inflammatory diseases or conditions, either of chronic or acute nature. Therefore, a further aspect of the present invention is also a method for the treatment of inflammatory diseases or allergic conditions, wherein the MCP-1 mutant protein according to the invention, the isolated polynucleic acid molecule or vector according to the present invention or a pharmaceutical preparation according to the invention is administered to a patient.

More specifically, the inflammatory diseases or allergic conditions are respiratory allergic diseases such as asthma, allergic rhinitis, COPD, hypersensitivity lung diseases, hypersensitivity pneumonitis, interstitial lung disease, (e.g. idiopathic pulmonary fibrosis, or associated with autoimmune diseases), anaphylaxis or hypersensitivity responses, drug allergies and insect sting allergies; inflammatory bowel diseases, such as Crohn's disease and ulcerative colitis; spondyloarthropathies, scleroderma; psoriasis and inflammatory dermatoses such as dermatitis, eczema, atopic dermatitis, allergic contact dermatitis, uticaria; vasculitis; autoimmune diseases with an aetiology including an inflammatory component such as arthritis (for example rheumatoid arthritis, arthritis chronica progrediente, psoriatic arthritis and arthritis deformans) and rheumatic diseases, including inflammatory conditions and rheumatic diseases involving bone loss, inflammatory pain, hypersensitivity (including both airways hypersensitivity and dermal hypersensitivity) and allergies. Specific auto-immune diseases include autoirmmune hematological disorders (including e.g. hemolytic anaemia, aplastic anaemia, pure red cell anaemia and idiopathic thrombocytopenia), systemic lupus erythromatosus, polychondritis, Wegener granulomatosis, dermatomyositis, chronic active hepatitis, myasthenia gravis, psoriasis, Steven-Johnson syndrome, autoimmune inflammatory bowel disease (including e.g. ulcerative colitis, Crohn's disease and Irritable Bowel Syndrome), autoimmune thyroiditis, Behcet's disease, endocrine ophthalmopathy, Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, and glomerulonephritis (with and without nephrotic syndrome, e. g. including idiopathic nephrotic syndrome or minimal change nephropathy); graft rejection (e.g. in transplantation including heart, lung, combined heart- lung, liver, kidney, pancreatic, skin, or corneal transplants) including allograft rejection or xenograft rejection or graft-versus-host disease, and organ transplant associated arteriosclerosis; atherosclerosis; cancer with leukocyte infiltration of the skin or organs; stenosis or restenosis of the vasculature, particularly of the arteries, e.g. the coronary artery, including stenosis or restenosis which results from vascular intervention, as well as neointimal hyperplasia; and other diseases or conditions involving inflammatory responses including ischemia reperfusion injury, hematologic malignancies, cytokine induced toxicity (e.g. septic shock or endotoxic shock), polymyositis, dermatomyositis, and granulomatous diseases including sarcoidosis

Preferably, the inflammatory disease is selected form the group comprising rheumatoid arthritis, uveitis, inflammatory bowel disease, myocardial infarction, congested heart failure or ischemia reperfusion injury.

The following examples describe the invention in more detail without limiting the scope of the invention.

## EXAMPLES

The carotide injury model as well as the animal models used for the present invention were performed in the laboratories of Prof. Christian Weber (Universitätsklinikum Aachen).

## Structural analysis of MCP-1 mutants upon GAG binding

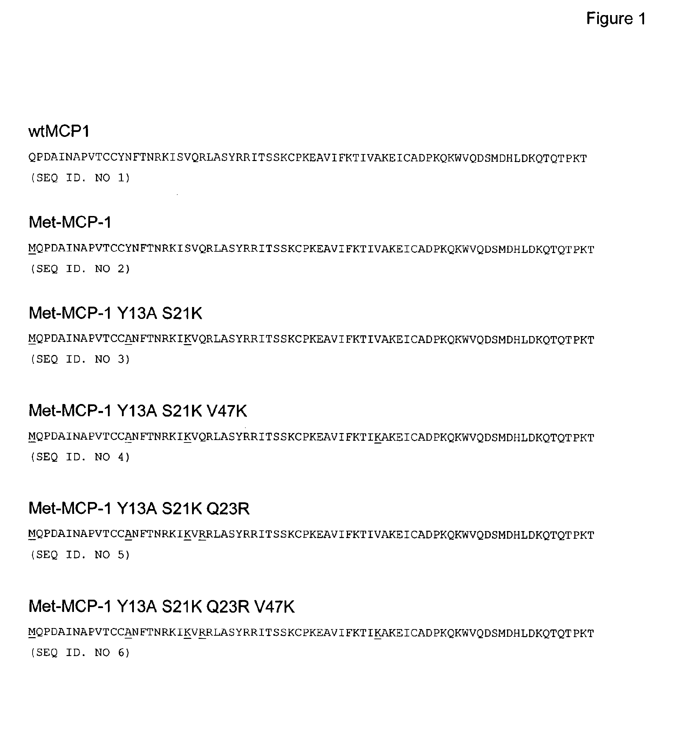

Analysis of secondary structural elements of MCP-1 mutants by far-UV CD spectroscopy showed that the overall ratio of alpha/beta/turns was conserved during protein design. Furthermore, protein unfolding studies showed that particularly Met-MCP-1 Y13AS21KQ23R exhibited very similar unfolding transition parameters compared to the wild type protein, indicating similar stability of these protein variants. Also the small secondary structural change induced by HS binding found for wtMCP-1 was reproduced in the MCP-1 mutants (as exemplified by the comparison of wtMCP-1 and Met-MCP-1 Y13AS21KQ23R in Figure 1). However, the stability of both proteins was significantly improved in the presence of HS as determined by temperature-induced unfolding studies. This means that contrary to other chemokines, HS impacts the fold of MCP-1 variants stronger than their secondary structure. This may be partly due to the elongated, partially unstructured form of MCP-1 in the absence of GAGs which experiences a structure-induction upon GAG binding, leading to a more compact fold and, thus, to greater stability.

## Increase in GAG binding affinity

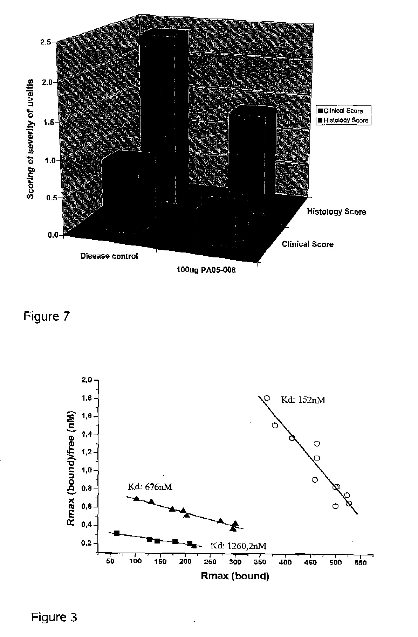

We have determined the increased GAG binding affinity by surface plasmon resonance (SPR) using a Biacore 3000 system. The immobilization of biotinylated HS onto a streptavidin coated CM4 sensor chip was performed according to an established protocol (28). The actual binding interactions were recorded at 25°C in PBS pH 7.4 containing 0.01% (v/v) P20 surfactant (BIAcore AB). 2.5 min injections of different protein concentrations at a flow rate of $60\mu l/min$ were followed by 5 min dissociation periods in buffer and a pulse of 1 M NaCl for complete regeneration. The maximum response signals of protein binding to the HS surface, corresponding to the plateaus of the respective sensograms, were used for Scatchard plot analysis and the calculation of equilibrium dissociation constants (Kd values). In Figure 3 the Scatchard plots of wtMCP-1 and two mutants are displayed. wtMCP-1 gave a Kd value of 1,26 $\mu$M, Met-MCP-1 Y13A S21 K yielded 676 nM, and Met-MCP-1 Y13A S21 K Q23R gave 152 nM. This means that in the latter mutant the affinity for HS has been improved by a factor of >8.

**Knock-out of GPCR activity**

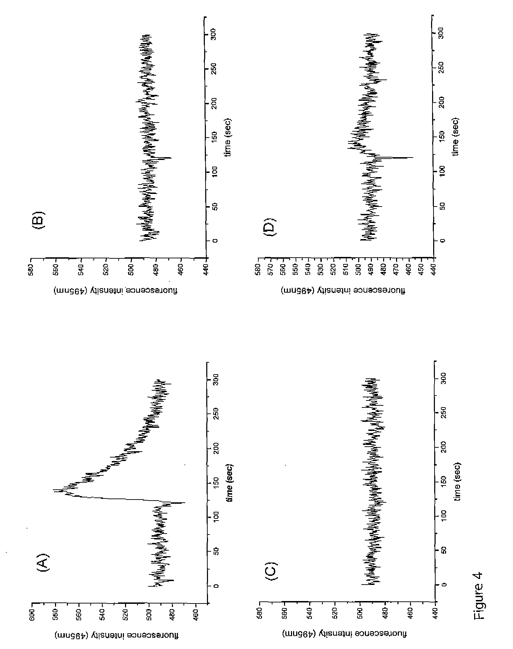

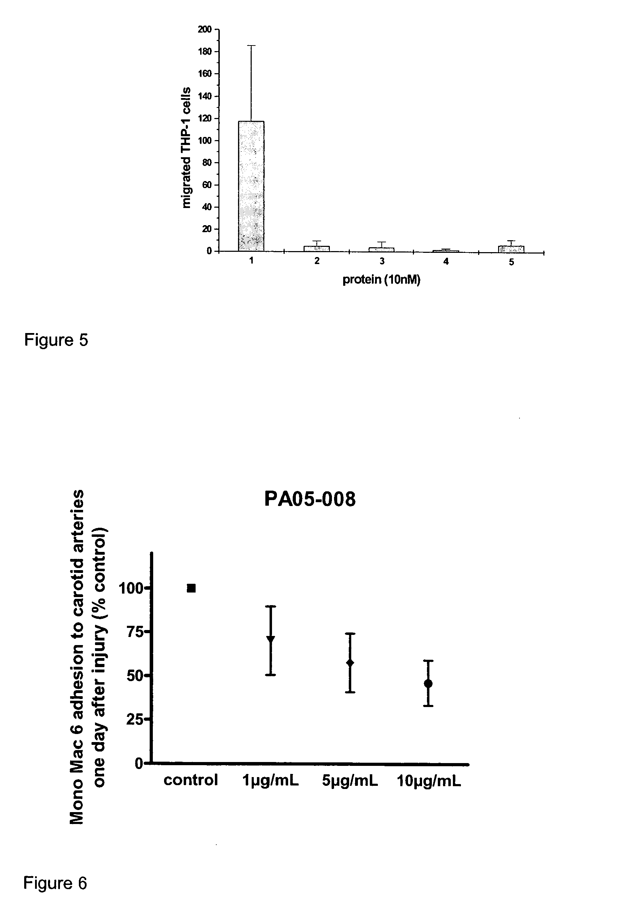

In order to obtain dominant-negative MCP-1 mutants, the GPCR activity of MCP-1 has been knocked out in addition to the improved GAG binding affinity. This was done by replacing the tyrosine residue at position 13 by an alanine residue and by keeping the N-terminal methionine residue. This led to a complete knock-out of MCP-1-related CCR2 activity, as exemplified by the complete absence of Ca influx and Thp-1 chemotaxis in the case of the Met-MCP-1 Y13A S21 K Q23R mutant (Figs. 4 & 5). The inability of this mutant to activate its high-affinity GPC receptor on target monocyte cells is expected to lead, in combination with the increased GAG binding affinity, to a potent inhibitor of MCP-1 activity in vivo.

**Inhibition of cell migration**

The effect of Met-MCP-1 Y13A S21 K Q23R on monocyte migration was investigated in an ex vivo model. For this purpose, apolipoprotein E-deficient (Apoe)-/- mice were subjected to wire-induced endothelial denudation injury after 1 week of atherogenic diet (1). After 24 hours carotid arteries were isolated for ex vivo perfusion as described (1). Carotid arteries were preperfused at 5 $\mu$L/min with Met-MCP-1 Y13A S21 K Q23R at a concentration of 1, 5 or 10 $\mu$g/mL for 30 min. Mono Mac 6 cells (1'10$^6$/mL) were labeled with calcein-AM, washed twice and perfused through the carotid artery. Adhesive interactions with the injured vessel wall were recorded using stroboscopic epifluorescence illumination (Drelloscop 250, Drello) and an Olympus BX51 microscope after 10 min of perfusion. By this means, a concentration dependent inhibition of monocyte adhesion by Met-MCP-1 Y13AS21 KQ23R was observed (see Figure 6).

**Inhibition/improvement of auto-immune uveitis**

Lewis rats were immunized into both hind legs with a total volume of 200 $\mu$l emulsion containing 15 $\mu$g PDSAg (retinal peptide) in complete Freund's adjuvant, fortified with Mycobacterium tuberculosis strain H37RA (BD, Heidelberg, Germany) to a final concentration of 2.5 mg/ml. 100 $\mu$g Met-MCP-1 Y13AS21 KQ23R mutant dissolved in 0.5 ml PBS (or PBS only as control) was applied i. p. daily from day 1 after active immunization until day 19. The time course of disease was determined by daily examination of animals with an ophthalmoscope. Uveitis was graded clinically as described (Gong J.H. and Clark-Lewis I., J. Exp.Med. 181 (2), 631-640 (1995))) and the average clinical score of all eyes is shown per group and day. As can be seen from Figure 7, the Met-MCP-1 Y13AS21 KQ23R mutant had a significant impact on the progression of the disease. Since uveitis is characterized by occular accumulation of T-cells and monocytes which finally lead to blindness, the therapeutic effect of Met-MCP-1 Y13AS21 KQ23R can be assigned to its inhibition of the migration of CCR2-activated leukocytes which mainly constitute monocytes and basophils.

**Inhibition/improvement of myocardial infarction**

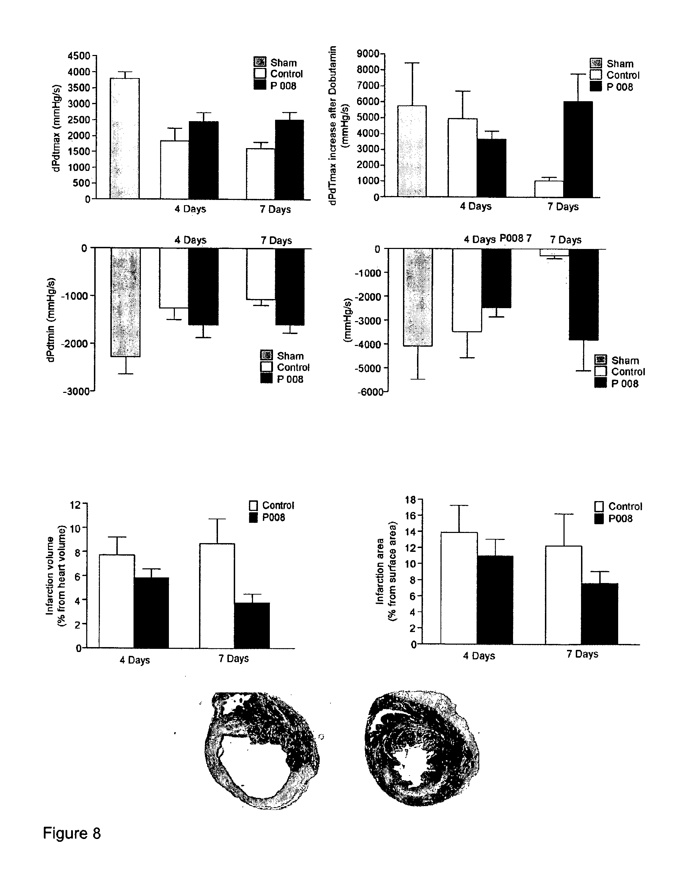

C57/B6 mice were intubated under general anaesthesia (100mg/kg ketamine and 10mg/kg xylasine, intraperitoneal) and positive pressure ventilation was maintained with oxygen and isofluran 0.2% using a rodent respirator. Hearts were exposed through a left toracotomy and MI was produced by suture occlusion of the left anterior descending artery (LAD) over a two mm silicon tube. The suture was opened after 30 min by cutting the silicon tube and reperfusion was re-established. In sham-operated mice, the suture was left open during the same time. The muscle layer and skin incision were closed with a silk suture. Animal experiments were approved by local authorities and complied with German animal protection law.
Met-MCP-1 Y13AS21KQ23R was dissolved in PBS at 100 $\mu$g/ml. Mice were treated intraperitoneally with 100 $\mu$l each during ischemia (10 min after ligation), 2 hours after reperfusion, and every day until the end point. Control mice were treated in the same way with vehicle.
At indicated time points, mice were anesthetized and the heart function was analyzed using a Langendorff apparatus (Hugo Sachs Elektronik-Harvard Apparatus) and HSE Isoheart software under constant perfusion pressure (100 mmHg) and electrical stimulation to assure a constant heart rate (600 bpm). The coronary flow, developed pressure, the increase (dP/dtmax) and decrease (dP/dtmin) in left ventricular pressure were measured without or with dobutamin (300 $\mu$mol in bolus). The measured parameters are displayed in Figure 8 (upper panel). At the end, the hearts were fixed in distension with 10% formalin and cut into 5 $\mu$m serial slices. Serial sections (10-12 per mouse, 400 $\mu$m apart, until mitral valve) were stained with Gomori's 1 step trichrome stain. The infarction area was determined on every section using Diskus software (Hilgers) and express as percent from total left ventricular volume (see Figure 8, lower panel).

**Claims**

1. MCP1 mutant protein with increased GAG binding affinity and reduced GPCR activity compared to wild type MCP-1 protein, **characterized in that** the MCP-1 protein is modified in a structure-conserving way by insertion of at least one basic and/or electron donating amino acid or replacement of at least one non-basic amino acid by at least one basic and/or electron donating amino acid and **in that** at least one amino acid of the first 1 to 10 amino acids of the N-terminal region of the wild type MCP-1 protein is modified by addition, deletion and/or replacement of at least one amino acid.

2. MCP-1 mutant protein according to claim 1, **characterized in that** modification in a structure conserving way is a deviation of the modified structure from wild type MCP1 structure of less than 30%, preferably less than 20% as measured by far-UV CD spectroscopy.

3. MCP-1 mutant protein according to claims 1 or 2, **characterized in that** the basic amino acids are selected from the group consisting of Arg, Lys, His.

4. MCP-1 mutant protein according to claims 1 or 2, **characterized in that** the electron donating amino acids are selected from the group consisting of Asn or Gln.

5. MCP-1 mutant protein according to any one of claims 1 to 4, **characterized in that** at least one amino acid at position 18, 24, 19, 49, 58 and/or 66 is modified.

6. MCP-1 mutant protein according to any one of claims 1 to 5, **characterized in that** at least one amino acid at position 21, 23 and/or 47 is modified.

7. MCP-1 mutant protein according to any one of claims 1 to 6, **characterized in that** Tyr at position 13 is substituted by Ala.

8. MCP-1 mutant protein of any of claims 1 to 7, containing an N-terminal Met.

9. MCP-1 mutant protein of any of claims 1 to 8, wherein the N-terminal amino acid residues 2-8 are deleted.

10. MCP-1 mutant protein, **characterized in that** it comprises the amino acid sequence of the general formula: $(M)_nQ$ $(PDAINAP)_m$VTCC(X1)NFTN RKI(X2)V(X3)RLAS YRRITSSKCP KEAVIFKTI(X4) AKEICADPKQ KWVQDSMDHL DKQTQTPKT wherein X1 is selected from the group consisting of Y and/or A, preferably it is A, wherein X2 is selected from the group consisting of S, R, K, H, N and/or Q, preferably it is K, wherein X3 is selected from the group consisting of R, K, H, N and/or Q, preferably it is R, wherein X4 is selected from the group consisting of V, R, K, H, N and/or Q, preferably it is K, and wherein n and/or m can be either 0 or 1.

11. MCP-1 mutant protein of any of claims 1 or 10, **characterized in that** it is selected from the group of Met-MCP-1 Y13A S21 K, Met-MCP-1 Y13A S21 K V47K, Met-MCP-1 Y13A S21 K Q23R and Met-MCP-1 Y13A S21 K Q23R V47K.

12. Isolated polynucleic acid molecule, **characterized in that** it codes for a protein according to any one of claims 1 to 11.

13. Isolated polynucleic acid molecule, **characterized in that** it hybridizes to the DNA molecule according to claim 12 under stringent conditions.

14. Vector, **characterized in that** it comprises an isolated DNA molecule according to claims 12 or 13.

15. Recombinant cell, **characterized in that** it is transfected with a vector according to claim 14.

16. Pharmaceutical composition, **characterized in that** it comprises a protein according to any one of claims 1 to 11, or a polynucleic acid molecule according to claims 12 or 13, or a vector according to claim 14, and a pharmaceutically acceptable carrier.

17. Use of MCP-1 mutant protein according to any one of claims 1 to 11, a polynucleic acid molecule according to claims 12 or 13, or a vector according to claim 14 in a method for in vitro inhibiting or suppressing the biological activity of the respective wild type protein.

18. Use of MCP-1 mutant protein according to any one of claims 1 to 11, a polynucleic acid molecule according to claims 12 or 13, or a vector according to claim 14, in a method for preparing a medicament for the treatment of a chronic or acute inflammatory disease or autoimmune conditions.

19. Use according to claim 18, **characterized in that** the inflammatory disease is selected from the group comprising rheumatoid arthritis, uveitis, inflammatory bowel disease, myocardial infarction, congested heart failure or ischemia reperfusion injury.

Figure 1

## wtMCP1

QPDAINAPVTCCYNFTNRKISVQRLASYRRITSSKCPKEAVIFKTIVAKEICADPKQKWVQDSMDHLDKQTQTPKT

(SEQ ID. NO 1)

## Met-MCP-1

MQPDAINAPVTCCYNFTNRKISVQRLASYRRITSSKCPKEAVIFKTIVAKEICADPKQKWVQDSMDHLDKQTQTPKT

(SEQ ID. NO 2)

## Met-MCP-1 Y13A S21K

MQPDAINAPVTCCANFTNRKIKVQRLASYRRITSSKCPKEAVIFKTIVAKEICADPKQKWVQDSMDHLDKQTQTPKT

(SEQ ID. NO 3)

## Met-MCP-1 Y13A S21K V47K

MQPDAINAPVTCCANFTNRKIKVQRLASYRRITSSKCPKEAVIFKTIKAKEICADPKQKWVQDSMDHLDKQTQTPKT

(SEQ ID. NO 4)

## Met-MCP-1 Y13A S21K Q23R

MQPDAINAPVTCCANFTNRKIKVRRLASYRRITSSKCPKEAVIFKTIVAKEICADPKQKWVQDSMDHLDKQTQTPKT

(SEQ ID. NO 5)

## Met-MCP-1 Y13A S21K Q23R V47K

MQPDAINAPVTCCANFTNRKIKVRRLASYRRITSSKCPKEAVIFKTIKAKEICADPKQKWVQDSMDHLDKQTQTPKT

(SEQ ID. NO 6)

Figure 2a

Figure 2b

Figure 4

Figure 5

Figure 6

Figure 7

Figure 3

Figure 8

**European Patent Office**

**EUROPEAN SEARCH REPORT**

Application Number

EP 07 45 0166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JARNAGIN KURT ET AL: "Identification of surface residues of the monocyte chemotactic protein 1 that affect signaling through the receptor CCR2" BIOCHEMISTRY, AMERICAN CHEMICAL SOCIETY. EASTON, PA, US, vol. 38, no. 49, 7 December 1999 (1999-12-07), pages 16167-16177, XP002336958 ISSN: 0006-2960 | 1-10, 12-17 | INV. C07K14/52 |
| Y | * abstract * | 18,19 | |
| Y | WO 2005/054285 A (KUNGL ANDREAS J [AT]) 16 June 2005 (2005-06-16) * page 3 - page 4; claims 2,3,5; table 1 * * page 7 - page 8 * * page 14, paragraph 2 * | 18,19 | |
| X | US 2003/162737 A1 (EGASHIRA KENSUKE [JP] ET AL) 28 August 2003 (2003-08-28) * the whole document * | 1-3,5,9, 10,12-19 | |
| X | STEITZ S A ET AL: "Mapping of MCP-1 functional domains by peptide analysis and site-directed mutagenesis" FEBS LETTERS, ELSEVIER, AMSTERDAM, NL, vol. 430, no. 3, 3 July 1998 (1998-07-03), pages 158-164, XP004258051 ISSN: 0014-5793 * the whole document * | 1,3,5,7, 10,12-17 | |

TECHNICAL FIELDS SEARCHED (IPC)

C07K

-/--

The present search report has been drawn up for all claims

| Place of search | Date of completion of the search | Examiner |
|---|---|---|
| Munich | 12 December 2007 | Rutz, Berthold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**European Patent Office**

Application Number

EP 07 45 0166

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PAAVOLA CHAD D ET AL: "Monomeric monocyte chemoattractant protein-1 (MCP-1) binds and activates the MCP-1 receptor CCR2B" JOURNAL OF BIOLOGICAL CHEMISTRY, AMERICAN SOCIETY OF BIOLOCHEMICAL BIOLOGISTS, BIRMINGHAM,, US, vol. 273, no. 50, 11 December 1998 (1998-12-11), pages 33157-33165, XP002283331 ISSN: 0021-9258 * the whole document * | 1,2,7, 10,12-17 | |
| A | POTZINGER H ET AL: "Developing chemokine mutants with improved proteoglycan affinity and knocked-out GPCR activity as anti-inflammatory recombinant drugs." BIOCHEMICAL SOCIETY TRANSACTIONS JUN 2006, vol. 34, no. Pt 3, June 2006 (2006-06), pages 435-437, XP002462147 ISSN: 0300-5127 | | |
| A | PROTAFFIN BIOTECHNOLOGIE AG: "CellJammerTM discovery technology"[Online] 7 September 2007 (2007-09-07), XP002462148 Retrieved from the Internet: URL:http://www.protaffin.com/images/conten t/pdfs/070907_protaffin_technology_profile .pdf?sid=05laac50gtpn2i073rg8k3mnk6> [retrieved on 2007-12-04] | | TECHNICAL FIELDS SEARCHED (IPC) |

-/--

The present search report has been drawn up for all claims

| Place of search | Date of completion of the search | Examiner |
|---|---|---|
| Munich | 12 December 2007 | Rutz, Berthold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................
& : member of the same patent family, corresponding document

EPO FORM 1503 03.82 (P04C01)

**European Patent Office**

**EUROPEAN SEARCH REPORT**

Application Number

EP 07 45 0166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GESSLBAUER BERND ET AL: "Glycomic approaches toward drug development: therapeutically exploring the glycosaminoglycanome." CURRENT OPINION IN MOLECULAR THERAPEUTICS DEC 2006, vol. 8, no. 6, December 2006 (2006-12), pages 521-528, XP002462149 ISSN: 1464-8431 ----- | | |
| D,A | LAU ELAINE K ET AL: "Identification of the glycosaminoglycan binding site of the CC chemokine, MCP-1 - Implications for structure and function in vivo" JOURNAL OF BIOLOGICAL CHEMISTRY, vol. 279, no. 21, 21 May 2004 (2004-05-21), pages 22294-22305, XP002462150 ISSN: 0021-9258 ----- | | |
| D,A | HEMMERICH STEFAN ET AL: "Identification of residues in the monocyte chemotactic protein-1 that contact the MCP-1 receptor, CCR2" BIOCHEMISTRY, AMERICAN CHEMICAL SOCIETY. EASTON, PA, US, vol. 38, no. 40, 5 October 1999 (1999-10-05), pages 13013-13025, XP002198634 ISSN: 0006-2960 ----- | | TECHNICAL FIELDS SEARCHED (IPC) |

The present search report has been drawn up for all claims

| Place of search | Date of completion of the search | Examiner |
|---|---|---|
| Munich | 12 December 2007 | Rutz, Berthold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 45 0166

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2007

| Patent document cited in search report | | Publication date | Patent family member(s) | | Publication date |
|---|---|---|---|---|---|
| WO 2005054285 | A | 16-06-2005 | AT | 412785 B | 25-07-2005 |
| | | | AT | 19522003 A | 15-12-2004 |
| | | | AU | 2004295104 A1 | 16-06-2005 |
| | | | BR | PI0416544 A | 09-01-2007 |
| | | | CA | 2546789 A1 | 16-06-2005 |
| | | | CN | 1890264 A | 03-01-2007 |
| | | | EP | 1689775 A1 | 16-08-2006 |
| | | | KR | 20070001917 A | 04-01-2007 |
| US 2003162737 | A1 | 28-08-2003 | AU | 5883601 A | 03-12-2001 |
| | | | WO | 0189582 A1 | 29-11-2001 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 05054285 A

### Non-patent literature cited in the description

- **BAGGIOLINI M.** *J. Int. Med.,* 2001, vol. 250, 91-104
- **GUPTA S.K. et al.** *Proc.Natl.Acad.Sci., USA,* 1995, vol. 92 (17), 7799-7803
- **ALI S. et al.** *Biochem.J.,* 2001, vol. 358, 737-745
- **LAU E.K. et al.** *J. Biol. Chem.,* 2004, vol. 279 (21), 22294-22305
- **NELKEN N.A. et al.** *J. Clin. Invest.,* 1991, vol. 88, 1121-1127
- **YLA-HERTTUALA, S.** *Proc. Natl. Acad. Sci USA,* 1991, vol. 88, 5252-5256
- **KOCH A.E. et al.** *J. Clin. Invest.,* 1992, vol. 90, 772-779
- **HOSAKA S. et al.** *Clin. Exp. Immunol.,* 1994, vol. 97 (3), 451-457
- **ROBINSON E. et al.** *Clin. Exp. Immunol.,* 1995, vol. 101 (3), 398-407
- **MACDERMOTT R.P. et al.** *J. Clin. Immunol.,* 1999, vol. 19, 266-272
- **AUKRUST P. et al.** *Circulation,* 1998, vol. 97, 1136-1143
- **HOHENSINNER P.J. et al.** *FEBS Letters,* 2006, vol. 580, 3532-3538
- **GREWAL I.S. et al.** *J. Immunol.,* 1997, vol. 159 (1), 401-408
- **BORING L. et al.** *J. Biol. Chem.,* 1996, vol. 271 (13), 7551-7558
- **KUZIEL W.A. et al.** *Proc.Natl.Acad.Sci. USA,* 1997, vol. 94 (22), 12053-8
- **LU B. et al.** *J. Exp.Med.,* 1998, vol. 187 (4), 601-8
- **LUKACS N.W. et al.** *J.Immunol.,* 1997, vol. 158 (9), 4398-4404
- **FLORY C.M. et al.** *1. Lab. Invest.,* 1993, vol. 69 (4), 396-404
- **GONG J.H. et al.** *J.Exp.Med.,* 1997, vol. 186 (1), 131-7
- **ZISMAN D.A. et al.** *J.Clin.Invest.,* 1997, vol. 99 (12), 2832-6
- **ALCAMI A. et al.** *J. Immunol.,* 1998, vol. 160 (2), 624-33
- **GOSLING J. et al.** *J. Clin. Invest.,* 1999, vol. 103 (6), 773-8
- **CHAD D. PAAVOLA et al.** *J. Biol. Chem.,* 1998, vol. 273 (50), 33157-33165
- **HEMMERICH S. et al.** *Biochemistry,* 1999, vol. 38 (40), 13013-13025
- **JARNAGIN K. et al.** *Biochemistry,* 1999, vol. 38, 16167-16177
- **SAMBROOK et al.** Molecular Cloning: A laboratory manual. Cold Spring Harbor, 1989
- **GONG J.H. ; CLARK-LEWIS I.** *J. Exp.Med.,* 1995, vol. 181 (2), 631-640

SEQUENCE LISTING

<110>   Protaffin Biotechnologie AG

<120>   Glycosaminoglycan-antagonising MCP-1 mutants and methods of using
        same

<130>   R 50771

<160>   7

<170>   PatentIn version 3.4

<210>   1
<211>   76
<212>   PRT
<213>   Homo sapiens

<400>   1

Gln Pro Asp Ala Ile Asn Ala Pro Val Thr Cys Cys Tyr Asn Phe Thr
1               5               10              15


Asn Arg Lys Ile Ser Val Gln Arg Leu Ala Ser Tyr Arg Arg Ile Thr
            20              25              30


Ser Ser Lys Cys Pro Lys Glu Ala Val Ile Phe Lys Thr Ile Val Ala
        35              40              45


Lys Glu Ile Cys Ala Asp Pro Lys Gln Lys Trp Val Gln Asp Ser Met
    50              55              60


Asp His Leu Asp Lys Gln Thr Gln Thr Pro Lys Thr
65              70              75


<210>   2
<211>   77
<212>   PRT
<213>   Artificial

<220>
<223>   modified MCP-1 protein

<400>   2

Met Gln Pro Asp Ala Ile Asn Ala Pro Val Thr Cys Cys Tyr Asn Phe
1               5               10              15


Thr Asn Arg Lys Ile Ser Val Gln Arg Leu Ala Ser Tyr Arg Arg Ile
            20              25              30


Thr Ser Ser Lys Cys Pro Lys Glu Ala Val Ile Phe Lys Thr Ile Val
        35              40              45


Ala Lys Glu Ile Cys Ala Asp Pro Lys Gln Lys Trp Val Gln Asp Ser
    50              55              60


Met Asp His Leu Asp Lys Gln Thr Gln Thr Pro Lys Thr
65              70              75

<210> 3
<211> 77
<212> PRT
<213> Artificial

<220>
<223> modified MCP-1 protein

<400> 3

Met Gln Pro Asp Ala Ile Asn Ala Pro Val Thr Cys Cys Ala Asn Phe
1               5                   10                  15

Thr Asn Arg Lys Ile Lys Val Gln Arg Leu Ala Ser Tyr Arg Arg Ile
            20                  25                  30

Thr Ser Ser Lys Cys Pro Lys Glu Ala Val Ile Phe Lys Thr Ile Val
            35                  40                  45

Ala Lys Glu Ile Cys Ala Asp Pro Lys Gln Lys Trp Val Gln Asp Ser
        50                  55                  60

Met Asp His Leu Asp Lys Gln Thr Gln Thr Pro Lys Thr
65                  70                  75

<210> 4
<211> 77
<212> PRT
<213> Artificial

<220>
<223> modified MCP-1 protein

<400> 4

Met Gln Pro Asp Ala Ile Asn Ala Pro Val Thr Cys Cys Ala Asn Phe
1               5                   10                  15

Thr Asn Arg Lys Ile Lys Val Gln Arg Leu Ala Ser Tyr Arg Arg Ile
            20                  25                  30

Thr Ser Ser Lys Cys Pro Lys Glu Ala Val Ile Phe Lys Thr Ile Lys
            35                  40                  45

Ala Lys Glu Ile Cys Ala Asp Pro Lys Gln Lys Trp Val Gln Asp Ser
        50                  55                  60

Met Asp His Leu Asp Lys Gln Thr Gln Thr Pro Lys Thr
65                  70                  75

<210> 5
<211> 77
<212> PRT
<213> Artificial

<220>
<223> modified MCP-1 protein

<400> 5

9

```
Met Gln Pro Asp Ala Ile Asn Ala Pro Val Thr Cys Cys Ala Asn Phe
1               5                   10                  15

Thr Asn Arg Lys Ile Lys Val Arg Arg Leu Ala Ser Tyr Arg Arg Ile
            20              25                  30

Thr Ser Ser Lys Cys Pro Lys Glu Ala Val Ile Phe Lys Thr Ile Val
        35              40                  45

Ala Lys Glu Ile Cys Ala Asp Pro Lys Gln Lys Trp Val Gln Asp Ser
    50              55                  60

Met Asp His Leu Asp Lys Gln Thr Gln Thr Pro Lys Thr
65                  70                  75
```

```
<210>   6
<211>   77
<212>   PRT
<213>   Artificial

<220>
<223>   modified MCP-1 protein

<400>   6
```

```
Met Gln Pro Asp Ala Ile Asn Ala Pro Val Thr Cys Cys Ala Asn Phe
1               5                   10                  15

Thr Asn Arg Lys Ile Lys Val Arg Arg Leu Ala Ser Tyr Arg Arg Ile
            20              25                  30

Thr Ser Ser Lys Cys Pro Lys Glu Ala Val Ile Phe Lys Thr Ile Lys
        35              40                  45

Ala Lys Glu Ile Cys Ala Asp Pro Lys Gln Lys Trp Val Gln Asp Ser
    50              55                  60

Met Asp His Leu Asp Lys Gln Thr Gln Thr Pro Lys Thr
65                  70                  75
```

```
<210>   7
<211>   77
<212>   PRT
<213>   Artificial

<220>
<223>   mutated MCP-1 protein


<220>
<221>   MISC_FEATURE
<222>   (1)..(1)
<223>   M = M or no amino acid residue

<220>
<221>   MISC_FEATURE
<222>   (3)..(9)
<223>   PDAINAP = PDAINAP or no amino acid residue

<220>
```

```
<221>  MISC_FEATURE
<222>  (14)..(14)
<223>  Xaa is Tyr or Ala

<220>
<221>  MISC_FEATURE
<222>  (22)..(22)
<223>  Xaa is Ser, Arg, Lys, His, Asn and/or Gln

<220>
<221>  MISC_FEATURE
<222>  (24)..(24)
<223>  Xaa is Arg, Lys, His, Asn and/or Gln

<220>
<221>  MISC_FEATURE
<222>  (48)..(48)
<223>  Xaa is Val, Arg, Lys, His, Asn and/or Gln

<400>  7

Met Gln Pro Asp Ala Ile Asn Ala Pro Val Thr Cys Cys Xaa Asn Phe
1               5               10              15


Thr Asn Arg Lys Ile Xaa Val Xaa Arg Leu Ala Ser Tyr Arg Arg Ile
        20              25              30


Thr Ser Ser Lys Cys Pro Lys Glu Ala Val Ile Phe Lys Thr Ile Xaa
        35              40              45


Ala Lys Glu Ile Cys Ala Asp Pro Lys Gln Lys Trp Val Gln Asp Ser
        50              55              60


Met Asp His Leu Asp Lys Gln Thr Gln Thr Pro Lys Thr
65              70              75
```